(19) Europäisches Patentamt
European Patent Office
Office européen des brevets

(11) Publication number: **0 256 643 A2**

(12) **EUROPEAN PATENT APPLICATION**

(21) Application number: 87305635.2

(22) Date of filing: 24.06.87

(51) Int. Cl.[4]: C08J 7/12 , C08K 5/54 , C07F 7/18 , A61L 31/00

(30) Priority: 11.07.86 GB 8616985

(43) Date of publication of application: 24.02.88 Bulletin 88/08

(84) Designated Contracting States: DE FR GB SE

(71) Applicant: Hugh Steeper Limited
237-239 Roehampton Lane
London SW15 4LB(GB)

(72) Inventor: Winter, Raymond William
7 Somerset View Sully
South Glamorgan CF1 6SZ Wales(GB)
Inventor: Barker, Nigel
12 Alfriston Avenue
West Croydon Surrey CR0 3DD(GB)

(74) Representative: Heath, Derek James et al
Bromhead & Co. 30 Cursitor Street Chancery Lane
London EC4A 1LT(GB)

(54) **Surface and Bulk Modification of plasticized materials.**

(57) A surface-modified or bulk-modified plasticized PVC or other plastics material with soil-resistant properties to make the material suitable for use inter alia as a prosthetic glove material. Preferably the plasticized material has a substantive layer of a silane bonded onto a surface thereof to give a soil-resistant coating to that surface.

EP 0 256 643 A2

## Surface and Bulk Modification of Plasticized Materials

This invention relates to surface and bulk modified plasticized material with soil-resistant properties to make the material suitable for use inter alia as a prosthetic glove material.

Current prosthetic gloves are made from PVC plastisols by casting into a female mould and following this with a heat treatment leading to gellation and curing. Such gloves suffer from a number of deficiencies, particularly poor resistance to soiling. Thus, the gloves readily take up ballpoint pen ink, newsprint and oleoginous contamination permanently, thereby giving them an unsightly appearance. This causes embarrassment to the wearer who is already severely conscious of his or her handicap.

According to the invention from one of its aspects, the above deficiency is substantially overcome by bonding a substantive layer of a silane on to a or the surface of plasticized PVC or other plasticized material to give a soil-resistant coating.

In the course of research on the surface treatment of PVC plastisols it was found that the treatment of plasticized PVC surfaces with selected silanes gave rise to surfaces with soil resistance. The silanes which conferred the best soil resistance were 3-aminopropyl triethyoxysilane [I] (Union Carbide A1100) and 3-aminopropyl-2-aminoethyl trimethoxysilane [II] (Union Carbide A1120, Dow Corning Z6020). When plasticized PVC was treated with these silanes the resulting surfaces could be soiled with ballpoint ink and cleaned completely with a simple alcohol wipe. The soiling and cleaning process could be repeated many times with minimal loss of film integrity.

It was at first thought that "activation" of the surface with dilute hydroxide solution to produce sites of unsaturation would be necessary. However, the use of $I^{125}$ labelled iodine monochloride indicated that the curing process used on the plastisols induced a sufficient level of unsaturation at the surface of the PVC and no further "activation" was necessary other than heating the glove.

The invention also extends to the bulk treatment of PVC plastisols by modification of PVC plasticizers, and this will now be described.

The low surface energy of silicones results in incompatibility when added directly to PVC-plasticizer systems. Thus, modification of the plasticizer is required to permit orientation of the silicone moiety at the glove surface in order to confer soil resistance.

The preparation of such plasticizers has been carried out using several routes as follows :

1. Copolymerisation of unsaturated plasticizers (maleates and fumarates) with vinyl silanes.
2. Preparation of a silyl amino alkyl benzoate from alkyl hydrogen phthalate and A1100. [I].
3. Direct aminolysis of phthalic anhydride and its derivatives..
4. Preparation of silyl pyrrolidones from aminosilanes and itaconate esters.

Of these, routes 1 and 2 proved to be disappointing but routes 3 and 4 showed some encouragement. In the case of route 3, infra-red analysis indicates that direct aminolysis of phthalate esters by aminosilanes will produce a silicone functional alkyl phthalate, but the rate of reaction is very slow. Aminolysis of phthalic anhydride by aminosilanes to produce "amic acids" has also been investigated. The amic acids [III, IV] produced were very reactive and tend to cure to solid materials. When incorporated into PVC plastisols they tended to cause bubbling in the sheet due to the release of alcohol. Although these materials may not be entirely suitable as plasticizers, their ability to cure rapidly may make them suitable for use as a surface coating on plasticized PVC in certain applications.

The reaction between primary alkyl amines and itaconate esters (Route 4) to produce alkyl pyrrolidine 4-carboxylates (pyrrolidones) is known in the literature. Alkyl pyrrolidine 4-carboxylates exhibit excellent plasticizing ability which is on a par with that of dialkyl phthalates. Two analogues of a simple alkyl pyrrolidine 4-carboxylate were produced by replacing the primary alkyl amine with either A1100 [I] or A1120 [II] to give n-butyl 2-oxo-1-[3-triethoxysilylpropyl]-pyrrolidine-4-carboxylate [V] and n-butyl-2-oxo-1-[2-[(3-trimethoxysilylproply) amino]ethyl] pyrrolidine-4-carboxylate [VI] respectively. Both materials showed excellent plasticizing properties and good compatibility with existing plasticizers. Soil retardancy tests have been carried out on plasticized PVC samples where 20% of the standard plasticizer has been replaced with the novel plasticizers. Both show some soil retardant properties, the sample containing the n-butyl 2-oxo-1-[2-[(3-trimethoxysilylpropyl)amino]ethyl] pyrrolidine-4-carboxylate [VI] giving the superior results.

A side effect of using n-butyl 2-oxo-1-[2-[(3-trimethoxysilylpropyl)amino]ethyl]-pyrrolidine-4-carboxylate [VI] as a co-plasticizer has appeared. During fabrication of PVC sheet, the PVC plastisol is cured in an oven at 180°C for 20 minutes. This has no adverse effects on the PVC but when a sample of the cured product is kept at 70°C for 48 hours discolouration and cross-linking occurs. This has been traced to the secondary amine present in the side chain of the platicizer abstracting HCl from the PVC molecules, degrading the PVC and forming n-butyl 2-oxo-1-[2-[(3-trimethoxysilylpropyl)amino]ethyl]pyrrolidine-4-carboxylate hydrochloride [VII].

The effect of the secondary amine has been neutralised by reacting it with suitable reagents to sequester the basic hydrogen which was causing the dehydrochlorination. Two modified forms were produced by reacting the free secondary amino group with reagents such as phenyl glycidyl ether [VIII] or 3-glycidoxypropyl trimethoxysilane (Dow Corning Z6040) [IX], each containing an epoxy group to give n-butyl 2-oxo-1-[2[(3-trimethoxysilylpropyl)-3'-(1-[1-trimethoxysilyl] propoxy-2-hydroxypropyl)] aminoethyl] pyrrolidine-4-carboxylate [X] and n-butyl 2-oxo-1-[2[(3-trimethoxysilylpropyl-3'(1-phenoxy-2-hydroxypropyl)) aminoethyl]pyrrolidine-4-carboxylate [XI]. When either of the modified plasticizers is incorporated into a PVC plastisol, no degradation occurs when the sample is kept at 70° C for 48 hours, unlike the unmodified silyl pyrrolidine-4-carboxylates [V, VI], and both modified plasticizers exhibit good plasticization properties and moreover soil resistance in cured films. However, the plasticizer molecule is now less stable at the cure temperature of PVC plastisols and the cleavage of an alcohol species from the plasticizer can result in the formation of bubbles in the film. The reduction in the high temperature stability of the plasticizers, [X and XI] occurred in the presence of a commercial calcium/zinc soap PVC heat stabilizer added to the PVC. When the calcium/zinc soap was replaced with a commercial thiotin soap, the stability of the epoxy modified plasticizers was improved.

A theoretical examination of the possible interactions between the two metal soaps and the epoxy modified silyl pyrrolidine-4-carboxylates [X, XI] gives no indication as to why the calcium/zinc soap destabilizes the plasticizer and the thiotin soap does not. An investigation into the method of preparation of the two commercial metal soap stabilizers indicates a large difference in the level of free acid present in the stabilizers, the level being very low in the case of the thiotin soap. From the literature it is known that the presence of either bases or acids can catalyse the cleavage of an alcohol from alkoxysilanes with the formation of silanols and therefore the level of free acid in the stabilizer is the most probable cause of the destabilization of the epoxy modified plasticizers [X, XI]. The free acid originates from the acid precursor used in making the stabilizer. To check this possibility, a sample of thiotin soap was treated with a very small amount of stearic acid before incorporation into a plastisol. Curing the plastisol resulted in a very badly bubbled sheet whereas the use of the pure thiotin gave no bubbling - thus confirming the literature findings.

In the preparation of prosthetic gloves, however, it is undesirable to use a thiotin soap because of its strong unpleasant odour. A commercial, low-acid, calcium/zinc stabilizer was therefore obtained to check for suitability. This gave the plastisol an improved stability at the cure temperature but still showed a tendency to bubble. As only a very low level of stabilizer is used in the preparation of the plastisol, in the order of 1%, it may be possible to purify a standard calcium/zinc soap to remove the free acid or reduce it to an acceptable level for use with epoxy modified silyl pyrrolidine-4-carboxylate plasticizers [X, XI]

On the whole, the most promising line of research has been in the surface treatment of plasticized PVC. This has shown that it is possible to bond a substantive layer of a silane on to the surface of plasticized PVC, with the integrity of the layer being unaffected by repeated cleansing. Based on the enhanced cleanliness of the gloves their life expectancy may be significantly increased.

The invention has been described above with particular reference to prosthetic gloves. It is to be understood, however, that it can be applied equally well to other prosthetic apparatus and to articles remote from that field of application. Further, it is applicable to materials other than plasticized PVC. Indeed, the invention is broadly concerned with the production of soil retardent surfaces on natural and synthetic high polymeric materials such as plastics, elastomers, or coatings thereof, and on fibres and fabrics which are exposed to environments contaminated by dust, dirt and unwanted extraneous matter. For example, the invention includes within its scope the surface improvement of vinyl coated wallpapers, flooring, headlinings and upholstery as well as prosthetic devices or appliances. This, in turn, leads to reduced frictional characteristics which may be of value during the insertion of catheters and like devices. See in this light, the aim of the invention can be stated to be the conversion of medium to high energy surfaces such as PVC to lower values of surface energy in order to reduce the tendency of soiling matter (which usually has a fairly

high value of surface energy) to adhere. To achieve this aim, the surface of the PVC is modified with a chemical treatment which lowers the energy of the surface, such as with a suitable silicone or by the addition of a suitable silicone additive or silicone containing material which may act as a plasticizer to the bulk of the PVC.

A suitable class of silicone for surface treatment, or as a precursor for the synthesis of silicone plasticizers, has the formula :

R‴ -[R″] - Si $R'_3$

wherein R′ is a monovalent hydrocarbonoxy such as methoxy, ethoxy, propoxy, isopropoxy, alkoxy-alkoxy $-O(CH_2)_n-O-(CH_2)_mH$ where n and m are integers and range from 1 to 3, or acetoxy; R″ is vinyl, or mono, di or tri methylene, or aminopropyl; and R‴ is zero or H or aminoethyl or glycidoxy.

Preferred compounds are vinyl tris (2 methoxy ethoxy silane), N-2 amino-ethyl-3-amino propyl trimethoxy silane [II] and gamma glycidoxy propyl trimethoxy silanes [IX].

For the production of novel silicone plasticizers or additives which confer soil retardant properties to the bulk of the polymer, use is made of an ester of an unsaturated acid of the general formula

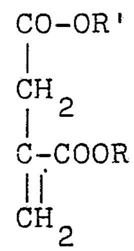

wherein R and R′ are groups chosen from methyl, ethyl, propyl, butyl, octyl or phenyl, to form a novel compound with a silane containing a primary or secondary amino group. where the product contains a secondary amino group, it may be advantageous to react that group with an epoxy functional compound.

The invention is illustrated by the following specific examples. Example 1 describes a standard test to characterise the susceptibility to staining. In the absence of the treatments described in the following examples, the test material, PVC, strongly absorbed the marking ink which showed signs of penetrating the sheet and diffising rapidly.

Example 1

Alcoholic solutions of 3 amino propyl triethoxy silane [I] ranging from 1-10% w/w were made. Into these solutions were dipped plasticized PVC sheets. The sheets were withdrawn after 10 minutes and allowed to dry and were then submitted to a standard soiling test in order to evaluate the level of stain resistance. The soiling test comprised marking the PVC with a black ballpoint pen and allowing the ink mark to remain for a period of 1 hour. The stained area was then wiped with an alcohol impregnated swab and examined for residual staining. If none was present, the PVC was marked again in the same area as before with the black pen. The protocol was repeated until the ink mark could not be removed by swabbing. If the number of repeats exceeded 20, the PVC was said to be soil resistant.

Example 2

Solutions ranging from 1-10% w/w in alcohol of N-2 aminoethyl-3 aminopropyl-trimethoxy silane [II] were prepared, and into these were dipped PVC plasticized sheets. These were withdrawn after 1 minute and dried. After 3 days they were marked as in Example 1. The soiling and cleansing process could be repeated in excess of 20 times without loss of film integrity or stain resistance.

Example 3

A number of plasticized PVC sheets were heat treated by placing them in an oven at 180° C for 2 minutes. On cooling, the sheets were dipped into alcoholic solutions of 3-aminopropyl-triethoxy silane [I] with concentrations ranging from 1-10% w/w as in Example 1. The sheets were withdrawn from the solutions and allowed to cure before applying the soiling/cleaning protocol described in Example 1. The sheets successfully passed the test.

Example 4

PVC prosthetic gloves were heat treated as in Example 3. Solutions of N-2-aminoethyl-3-amino-propyl-trimethoxy silane [II] in alcohol ranging from 1-10% w/w were prepared and the heat-treated gloves were then swabbed with these solutions. After 3 days the soiling/cleansing procedure was applied as in Example 1 and could be repeated in excess of 20 times.

Example 5

A 5% w/w solution of N-2-aminoethyl-3-aminopropyl trimethoxy silane [II] in alcohol was made and a plasticized PVC glove was heat-treated at 180° C for 2 minutes. On cooling, the grove was swabbed with the solution with the aid of cotton wool. After curing, the glove was deliberately soiled as in Example 1. No staining occurred after repeated marking with black ink.

Example 6

A plastisol was prepared from a vinyl chloride-vinyl acetate copolymer using the following formulation: Vestolit E 7091 (Huls) 100 pbw; di-octyl phthalate 100 pbw, Stanclere T483 1pbw. The formulation was cast and cured in the form of a sheet. A solution in alcohol of 5% w/w 3-aminopropyl-triethoxy [I] silane was prepared. The copolymer sheet was immersed in the solution for 1 minute. The sheet was removed, allowed to dry and, after 3 days, the standard marking test was applied. The sheet successfully passed the test.

Example 7

A silicone functional additive serving as a co-plasticizer for PVC was made by reacting 0.5 mole di n-butyl itaconate with 0.5 mole of N 2-amino ethyl 3 amino propyl trimethoxy [II] silane at room temperature. The system was vacuum distilled to remove the butan-1-ol by product. The product, n-butyl-2-oxo-1-[2-(3 methoxy silyl propyl) amino ethyl] pyrrolidine-4-carboxylate [VI] was a pale straw liquid. A PVC plastisol consisting of Breon 111 EP 100 pbw; di-octyl phthalate 80 pbw; n-butyl-2-oxo-1-[2-(3 methoxy silyl propyl) amino ethyl] pyrrolidine-4-carboxylate [VI] 20-pbw, Stanclere T483 1 pbw. The mixture was cast as a sheet, gelled and cured at 180 C for 26 minutes and easily passed the standard soiling test described in Example 1.

Example 8

An adduct of n-butyl-2-oxo-1-[2-(3-methoxysilyl propyl) aminoethyl]-pyrrolidine-4-carboxylate [VI] was prepared by reacting the pyrrolidine-4-carboxylate [VI] in equimolar proportions with phenyl glycidyl ether [VIII] at room temperature giving a yield in excess of 80%. A plastisol formlation using 15 pbw at the adduct [XI] was prepared as in Example 7. The PVC sheet thus prepared successfully passed the soiling/cleansing test given in Example 1.

Example 9

To the pyrrolidine-4-carboxylate [VI] (0.5 mole) described in Example 7 was slowly added 0.5 mole 3-glycidoxy-propyl timethoxy silane [IX] at room temperature giving a yield of the adduct in excess of 90%. A plastisol formulation was prepared as in Example 7 but using only 15 pbw of the adduct [X]. After gelling and curing, the PVC sheet showed excellent soil retardant properties showing that the adduct conferred anti-soiling characteristics which were not lost on repeated marking and cleaning cycles.

Example 10

A PVC sheet was prepared as in Example 9 using 10 pbw of the adduct [X]. After cure, the PVC sheet was allowed to cool before being dipped into a 5% w/w solution of N-2-aminoethyl-3-aminopropyl trimethoxy silane [III] in alcohol. On removal from the silane solution and subsequent curing of the film, the soiling/cleansing protocol was applied as in Example 1. The sheet easily passed the test.

Appendix: Formulae

I $NH_2(CH_2)_3Si(OC_2H_5)_3$
II $NH_2(CH_2)_3NH(CH_2)_2Si(OCH_3)_3$
III $(C_2H_5O)_3Si(CH_2)_3NHCO.C_6H_4.COOH$
IV $(CH_3O)_3Si(CH_2)_3NH(CH_2)_2NHCO.C_6H_4.COOH$

V $CH_2.CH(CO.OC_4H_9(CH_2.CO.N(CH_2)_3Si(OC_2H_5)_3$

VI $CH_2\ CH(CO.OC_4H_9)CH_2.CO.N(CH_2)_3NH(CH_2)_2Si(OCH_3)_3$

VII $CH_2.CH(CO.OC_4H_9)CH_2.CO.N(CH_2)_3NH(CH_2)_2Si(OCH_3)_3.HCl$

VIII $CH_2.CH.O.OCH_2O.C_6H_5$

IX $CH_2.CH.O.O(CH_2)_3Si(OCH_3)_3$

X $CH_2.CH(CO.OC_4H_9(CH_2.C).N(CH_2)_3N(CH_2)_2Si(OCH_3)_3$
$CH(CHOH).O(CH_2)_3Si(OCH_3)_3$

XI $CH_2.CH(CO.OC_4H_9)CH_2.CO.N(CH_2)_3N(CH_2)_2Si(OCH_3)_3$
$[CH_2.(CHOH).OC_6H_5]$

**Claims**

1. A method of surface treatment of plastics material, characterised in that the treatment is effected with at least one compound of the general formula :
R‴ -[R″] - Si R′$_3$
wherein

R′ is a monovalent hydrocarbonoxy such as methoxy, ethoxy, propoxy, isopropoxy, alkoxy-alkoxy $-O(CH_2)_n-O-(CH_2)_mH$ where n and m are integers and range from 1 to 3, or acetoxy;
R″ is vinyl, mono, di, or tri methylene, or aminopropyl; and
R‴ is zero, H, aminoethyl or glycidoxy.

2. A method according to claim 1, characterised in that the compound is vinyl tris (2-methoxy ethoxy-silane), N-2 aminoethyl-3-aminopropyl trimethoxy silane or a gamma glycidoxy-propyl-trimethoxy silane.

3. A method of producing novel silicone-containing plasticizers which confer soil-retardant properties to the bulk of the polymer characterised by treating an ester of an unsaturated acid of the general formula :

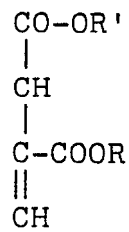

wherein
R and R′ are methyl, ethyl, propyl, butyl, octyl or phenyl,
to form a novel compound with a silane containing an amino group.

4. A method according to claim 3, characterised in that an alkyl pyrrolidine-4-carboxylate formed in the method is subsequently reacted with glycidyl ether containing molecules to confer anti-soiling characteristics.

5. A plasticized material, especially for making into prosthetic gloves, characterised by a substantive layer of a silane bonded onto a surface thereof to give a soil-resistant coating to that surface.

6. A plasticized material according to claim 5, characterised in that the silane is 3-aminopropyl triethyoxysilane.

7. A plasticized material according to claim 5, characterised in that the silaneis 3-aminopropyl-2-aminoethyl trimethoxy-silane.

8. A plasticized material according to claim 1, characterised in that the plastics material is PVC.